(19) Europäisches Patentamt
European Patent Office
Office européen des brevets

(11) Numéro de publication: **0 103 497**
**B1**

(12) **FASCICULE DE BREVET EUROPEEN**

(45) Date de publication du fascicule du brevet: **29.01.86**

(21) Numéro de dépôt: **83401518.2**

(22) Date de dépôt: **25.07.83**

(51) Int. Cl.⁴: **C 07 D 473/06**, C 07 D 473/08, C 07 D 473/10, C 07 D 473/12, A 61 K 31/52 // C07D239/54

# (54) Nouveaux dérivés de la xanthine, leur procédé de préparation et les compositions pharmaceutiques les renfermant.

(30) Priorité: **28.07.82 FR 8213155**

(43) Date de publication de la demande: **21.03.84 Bulletin 84/12**

(45) Mention de la délivrance du brevet: **29.01.86 Bulletin 86/5**

(84) Etats contractants désignés: **AT BE CH DE FR GB IT LI LU NL SE**

(56) Documents cités:
**EP - A - 0 023 350**
**CH - A - 409 974**
**FR - A - 2 489 331**
**FR - A - 2 514 003**
**FR - M - 5 086**
**FR - M - 7 828**

**Le dossier contient des informations techniques présentées postérieurement au dépôt de la demande et ne figurant pas dans le présent fascicule.**

(73) Titulaire: **ADIR, 22, rue Garnier, F-92200 Neuilly-sur-Seine (FR)**

(72) Inventeur: **Regnier, Gilbert, Avenue du Plessis, F-92290 Châtenay-Malabry (FR)**
Inventeur: **Guillonneau, Claude, 53 Rue de la Gaité, F-92140 Clamart (FR)**
Inventeur: **Duhault, Jacques, 14 Bis rue Paul Demange, F-78290 Croissy-sur-Seine (FR)**
Inventeur: **Boulanger, Michelle, "Le Val Cort" Escalier 6 71 Avenue A. Renoir, F-78160 Marly-le-Roi (FR)**

(74) Mandataire: **Reverbori, Marcelle et al, ADIR 22 Rue Garnier, F-92200 Neuilly-sur-Seine (FR)**

**EP 0 103 497 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés de la xanthine, leur procédé de préparation et les compositions pharmaceutiques les renfermant.

Elle concerne particulièrement les dérivés de la xanthine de formule générale I:

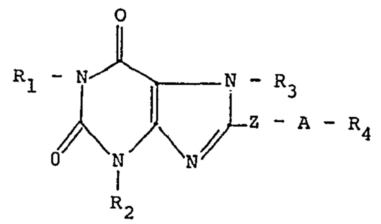

(I)

dans laquelle:

$R_1$ représente un atome d'hydrogène ou un radical alkyle en chaîne droite ou ramifiée, renfermant de 1 à 5 atomes de carbone;

$R_2$ représente un radical hydrocarboné en chaîne droite ou ramifiée renfermant jusqu'à 6 atomes de carbone, et comportant éventuellement une double liaison, un radical phényle, ou un radical benzyle;

$R_3$ représente un atome d'hydrogène ou un radical alkyle, hydroxyalkyle ou dihydroxyalkyle renfermant chacun de 1 à 5 atomes de carbone;

$R_4$ représente un groupe de formule:

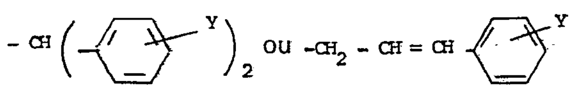

dans lesquelles:

Y représente un atome d'hydrogène ou d'halogène ou un radical alkyle ou alcoxy renfermant chacun de 1 à 5 atomes de carbone, ou un radical hydroxyle;

Z représente un radical méthylène ou un radical hydroxycarboné en chaîne droite ayant de 2 à 5 atomes de carbone éventuellement substitué par un radical hydroxyle, et

A représente un reste aminé de type:

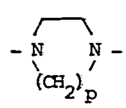

dans lequel p prend les valeurs 2 ou 3, ou

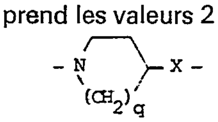

dans lequel:

q prend les valeurs 1 ou 2, et

X représente une liaison simple, un atome d'oxygène ou un groupe

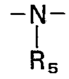

dans lequel:

$R_5$ représente un atome d'hydrogène ou un radical alkyle ou alkylène ayant chacun de 1 à 5 atomes de carbone, ou

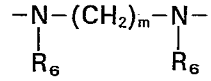

dans lequel:

m représente un nombre entier de 2 à 6, et

$R_6$ représente un radical alkyle renfermant de 1 à 5 atomes de carbone.

L'état antérieur de la technique dans ce domaine est illustré par le brevet FR-A N° 2489331 qui divulgue des dérivés de la théophylline substitués en position 7 par des radicaux (diphénylméthyl-4 pipérazinyl)-2 alkyl, ayant principalement des propriétés vasodilatatrices très importantes, mais d'une durée d'action brève. La titulaire a maintenant découvert des dérivés de la xanthine substitués notamment en position 8, dépourvus d'activité sur le système cardio-vasculaire, mais possédant une activité bronchodilatatrice et une durée d'action très importante.

La présente invention a également pour objet le procédé de préparation des dérivés de formule générale I, caractérisé en ce que:

l'on condense un dérivé halogéné de formule générale II:

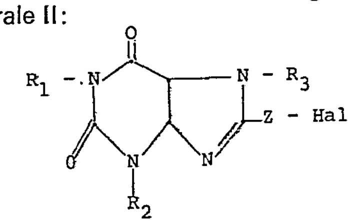

(II)

dans laquelle:

$R_1$, $R_2$, $R_3$ et Z ont les significations précédemment définies, et

Hal représente un atome de chlore ou de brome, avec un dérivé aminé de formule générale III:

$$H-A-R_4 \qquad (III)$$

dans laquelle:

A et $R_4$ sont tels que précédemment définis.

La condensation s'effectue de préférence dans un solvant choisi parmi les alcools renfermant jusqu'à 5 atomes de carbone, tel que par exemple le méthanol, l'éthanol, le propanol ou le butanol. Il est avantageux d'opérer à une température comprise entre 64 et 130° C en présence d'un accepteur de l'hydracide formé au cours de la réaction. Cet accepteur peut être choisi parmi les carbonates alcalins tels que les carbonates de sodium et de potassium, les amines tertiaires telles que la triéthylamine ou un excès du dérivé aminé de formule III utilisé pour la réaction.

Les matières premières de formule générale II ont été préparées selon le schéma opératoire suivant:

(schéma *en tête de la page suivante)*

Les matières premières de formule générale III sont des produits connus.

La présente invention a également pour objet le procédé de préparation des dérivés I répondant à la formule générale I':

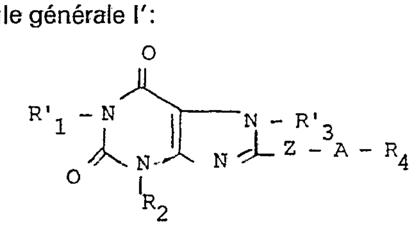

(I')

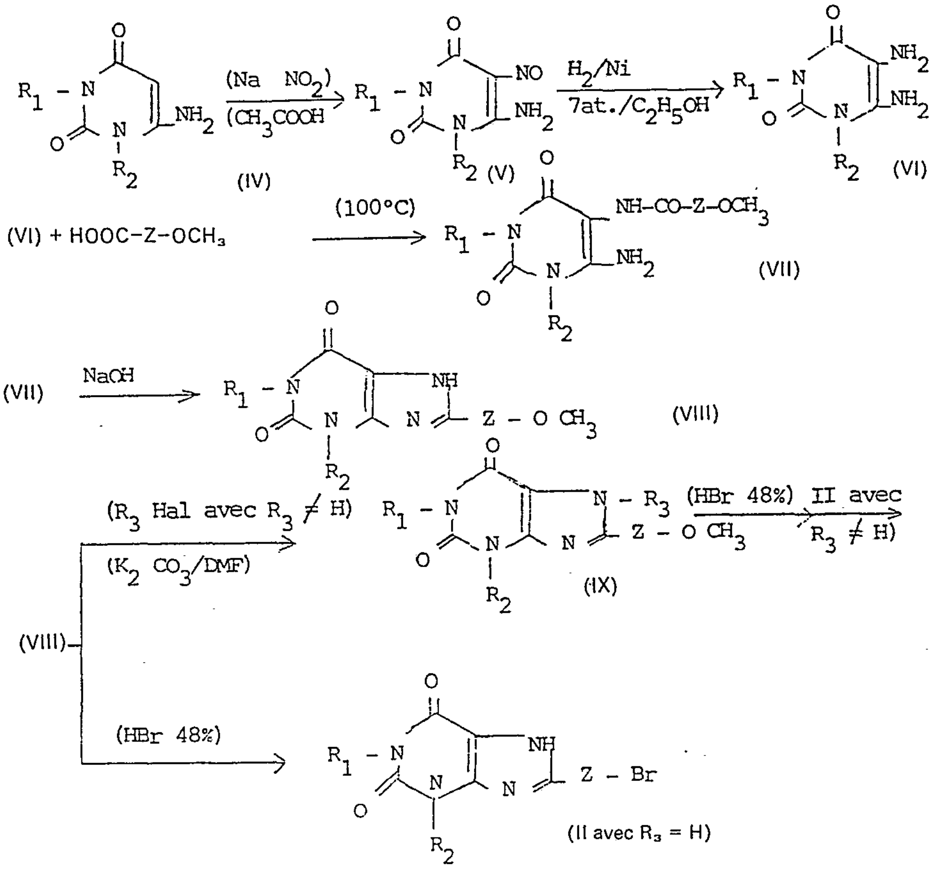


dans laquelle:

$R_2$, $R_4$, Z et A ont les significations précédemment définies;

$R'_1$ représente un radical alkyle en chaîne droite ou ramifiée, renfermant de 1 à 5 atomes de carbone, et

$R'_3$ représente un radical alkyle, hydroxyalkyle ou dihydroxyalkyle contenant chacun de 1 à 5 atomes de carbone,

caractérisé en ce que l'on condense un dérivé de formule II':

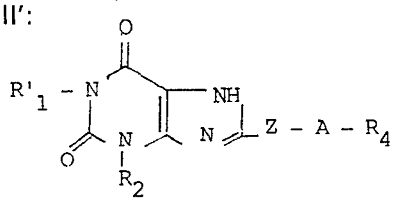
(II')

dans laquelle:

$R'_1$, $R_2$, $R_4$, Z et A ont les significations précédemment définies,

avec un dérivé halogéné de formule III':

$$R'_3-X \qquad (III')$$

dans laquelle:

$R'_3$ a la signification énoncée précédemment, et

X représente un atome de chlore ou de brome.

La condensation s'effectue de préférence dans un solvant adéquat tel que par exemple le diméthylformamide à une température comprise entre 80 et 120°C, en présence d'un accepteur de l'hydracide formé au cours de la réaction. Cet accepteur peut être, entre autres, un carbonate alcalin comme par exemple le carbonate de sodium ou de potassium.

Les nouveaux dérivés de formule générale I peuvent être transformés en sels d'addition avec les acides, sels qui font à ce titre partie de l'invention. Comme acides utilisables pour la formation de ces sels, on peut citer par exemple dans la série minérale: les acides chlorhydrique, bromhydrique, sulfurique, phosphorique, et dans la série organique: les acides acétique, propionique, maléique, fumarique, tartrique, citrique, oxalique, benzoïque, méthane sulfonique et iséthionique.

Ces nouveaux dérivés peuvent être purifiés par des méthodes physiques telles que cristallisation, chromatographie ou chimiques telles que formation de sels d'addition avec des acides et décomposition de ces sels par les agents alcalins.

Les dérivés de formule générale I et leurs sels physiologiquement tolérables possèdent des propriétés pharmacologiques et thérapeutiques intéressantes, notamment des propriétés bronchodilatatrice, anti-allergique et inhibitrice de la phosphodiestérase.

Leur toxicité est faible et leur $DL_{50}$ déterminée chez la souris est supérieure à 100 mg/kg par voie intrapéritonéale, et supérieure à 800 mg/kg par voie orale.

L'activité bronchodilatatrice a été étudiée chez le cobaye par la méthode de H. Konzett et R. Rossler, Arch. Exp. U. Pharm. 195, 71 (1940). On a ainsi observé que les dérivés de la présente invention injectés par voie intraveineuse à des doses variant, selon les dérivés, de 1 à 5 mg/kg inhibent totalement le bronchospasme provoqué par l'administration intraveineuse soit d'histamine, soit de sérotonine, et partiellement l'effet de l'acétylcholine et de la Slow Reacting Substance.

Soumis au test de A.K. Armitage, Brit. J. Pharmacol. 17, 196 (1961), les dérivés de l'invention administrés par voie orale à des doses variant de 0,5 à 10 mg/kg selon les composés, inhibent de 50% l'effet produit chez le cobaye par un aérosol d'histamine à 4%. Pour certains de ces composés, l'effet est encore très important 48 h après l'administration orale.

En outre, certains composés de l'invention possèdent un effet agoniste spécifique vis-à-vis des récepteurs purinergiques centraux et périphériques de type A1 et/ou A2 qui peut conduire à une spécificité d'effet thérapeutique.

A titre d'exemple non limitatif, l'administration orale de 5 mg/kg du composé de l'exemple 1 inhibe, pendant plus de 48 h, le bronchospasme provoqué par un aérosol d'histamine à 4%. Par ailleurs, une diminution de la réaction cutanée anaphylactique est observée chez le rat après l'administration unique de 20 mg/kg *per os* de ce composé.

Ce composé de l'exemple 1 est plus fortement agoniste des récepteurs A2 (IC 60 = 15 μM) que des récepteurs A1 (IC 50 > 100 μM).

Les propriétés pharmacologiques ci-dessus décrites, ainsi que la faible toxicité des dérivés de formule générale I et de leurs sels physiologiquement tolérables permettent leur utilisation en thérapeutique, notamment dans le traitement de toutes les maladies où il est nécessaire d'inhiber les réactions antigène-anticorps comme les maladies auto-immunes, allergiques et inflammatoires et en particulier celles dans lesquelles un effet bronchodilatateur est bienvenu telles que la dyspnée asthmatique et les bronchopneumopathie chroniques obstructives, en particulier à forme spastique. La longue durée d'action permet un traitement de crise aussi bien qu'un traitement de fond de la maladie asthmatique simple ou intriguée.

Par ailleurs, les propriétés spasmolytiques sont une indication dans le traitement des coliques néphrétiques ou hépatiques.

La présente invention a également pour objet les compositions pharmaceutiques contenant comme principe actif un dérivé de formule générale I, ou un de ses sels physiologiquement tolérables, mélangé ou associé à un excipient pharmaceutique approprié. Elle concerne notamment les formes dosées renfermant de 25 à 250 mg de principe actif.

Les compositions pharmaceutiques ainsi obtenues, sont présentées avantageusement sous des formes dosées diverses telles que par exemple comprimés, dragées, gélules, glosettes ou préparations galéniques appropriées pour une administration sublinguale, suppositoires, solutions injectables ou buvables, ainsi que sous des formes adaptées à l'administration par aérosol. Elles peuvent être administrées par la voie orale, rectale ou parentérale, aux doses de 25 à 250 mg de principe actif, 1 à 2 fois par jour.

Les exemples suivants, donnés à titre non limitatif, illustrent l'invention. Les points de fusion, sauf mention contraire, ont été déterminés à la platine chauffante de Kofler.

*Exemple 1:*

*Méthyl-1 isobutyl-3 [(diphénylméthyl-4 pipérazinyl)-2 éthyl]-8 xanthine*

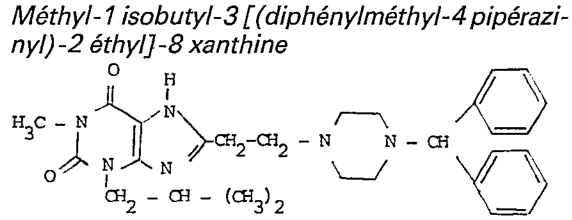

Une suspension de 12,2 g de méthyl-1 isobutyl-3 bromoéthyl-8 xanthine et de 23 g de benzhydryl-1 pipérazine dans 200 ml d'éthanol est chauffée à reflux. On observe une dissolution progressive et on chauffe ainsi pendant 20 à 24 h. On évapore ensuite à sec et reprend le résidu par une solution à 10% de bicarbonate de sodium. On extrait plusieurs fois avec $CH_2Cl_2$, sèche sur $Na_2SO_4$ et concentre à sec. Le résidu huileux est purifié par chromatographie flash sur 1 kg de silice 0,04-0,063 mm, en éluant avec de l'acétate d'éthyle puis avec un mélange d'acétate d'éthyle/méthanol (95/5). Après fractionnement et évaporation des éluats, on recueille 14 g de produit pur fondant à 200-202°C.

La méthyl-1 isobutyl-3 bromoéthyl-8 xanthine de départ, fondant à 210°C, a été préparée par bromuration avec HBr à 48% du dérivé méthoxyéthyl-8 correspondant, fondant à 163°C, lui-même préparé par cyclisation avec NaOH de l'isobutyl-1 méthyl-3 dioxo-2,4 tétrahydro-1,2,3,4 (méthoxy-3 propionamido)-5 amino-6 pyrimidine fondant à 200°C, elle-même préparée par condensation de l'acide méthoxy-3 propionique avec la diamino-5,6 isobutyl-1 méthyl-3 dioxo-2,4 tétrahydro-1,2,3,4 pyrimidine, elle-même préparée par réduction, en présence de nickel de Raney comme catalyseur, sous une pression d'hydrogène de six atmosphères, du dérivé nitroso-5 correspondant fondant à 228°C, lui-même préparé par nitrosation avec $NaNO_2/CH_3COOH$ de l'isobutyl-1 méthyl-3 dioxo-2,4 amino-6 tétrahydro-1,2,3,4 pyrimidine.

*Exemples 2 à 30:*

Les dérivés suivants ont été préparés selon le procédé décrit dans l'exemple 1:

2. La diméthyl-1,3 (diphénylméthyl-4 pipérazinyl méthyl)-8 xanthine, P.F.: 241°C (méthanol).

3. La diméthyl-1,3 [(diphénylméthyl-4 pipérazinyl)-2 éthyl]-8 xanthine, P.F. (capillaire): 103-106°C (chlorure de méthylène).

4. La diméthyl-1,3 [(diphénylméthyl-4 pipérazinyl)-3 propyl]-8 xanthine, P.F. (capillaire) du dichlorhydrate hémihydrate correspondant: 249-250°C (méthanol).

5. La méthyl-1 isobutyl-3 [(diphénylméthyl-4 pipérazinyl)-3 propyl]-8 xanthine, P.F. (capillaire) du dichlorhydrate monohydrate correspondant: 217-220°C (éthanol).

6. La triméthyl-1,3,7 [(diphénylméthyl-4 pipérazinyl)-3 propyl]-8 xanthine, P.F. du fumarate correspondant: 198°C (éthanol).

7. La diméthyl-1,7 isobutyl-3 [(diphénylméthyl-4 pipérazinyl)-3 propyl]-8 xanthine, P.F. du fumarate correspondant: 182°C (éthanol/éther).

8. La diméthyl-1,7 isobutyl-3 [(diphénylméthyl-4 pipérazinyl)-2 éthyl]-8 xanthine, P.F. (capillaire) du dichlorhydrate correspondant: 214-218°C (n. propanol/éther).

9. La dimétyl-1,7 phényl-3 [(diphénylméthyl-4 pipérazinyl)-3 propyl]-8 xanthine, P.F.: 150°C (isopropanol).

10. La méthyl-1 isobuyl-3 [(diparafluorophénylméthyl-4 pipérazinyl)-2 éthyl]-8 xanthine, P.F.: 184°C (acétate d'éthyle).

11. La diméthyl-1,7 isobutyl-3 [(diparafluorophénylméthyl-4 pipérazinyl)-2 éthyl]-8 xanthine, P.F. du dimaléate correspondant: 174°C (n. propanol).

12. La diméthyl-1,7 isobutyl-3 {[(diparafluorophénylméthyl)-4 pipérazinyl]-3 propyl}-8 xanthine, P.F. du fumarate correspondant: 180°C (éthanol).

13. La méthyl-1 isobutyl-3 {[N-(N'-diphénylméthyl N'-éthylamino)-2 éthyl] N-éthylamino-2 éthyl}-8 xanthine, P.F. (capillaire) du dichlorhydrate correspondant: 135-140°C (isopropanol/éther).

14. La diméthyl-1,7 isobutyl-3 {[N-(N'-diphénylméthyl N'-éthylamino)-2 éthyl] N-éthylamino-2 éthyl}-8 xanthine, P.F. (capillaire) du dichlorhydrate correspondant: 130-140°C (isopropanol/éther).

15. La diméthyl-1,7 isobutyl-3 {[N-(N'-diphénylméthyl N'-éthylamino)-2 éthyl] N-éthylamino-3 propyl}-8 xanthine, P.F. (capillaire) du dichlorhydrate correspondant: 125-135°C.

16. La diméthyl-1,7 n. propyl-3 [(diphénylméthyl-4 pipérazinyl)-3 propyl]-8 xanthine, P.F. du fumarate correspondant: 200°C (éthanol).

17. La méthyl-1 isobutyl-3 [(diphénylméthyloxy-4 pipéridino)-2 éthyl]-8 xanthine, P.F. (capillaire) du dichlorhydrate correspondant: 163-167°C (isopropanol/éther).

18. La diméthyl-1,7 isobutyl-3 [(diphénylméthyloxy-4 pipéridino)-2 éthyl]-8 xanthine, P.F. (capillaire) du fumarate correspondant: 173-177°C (n. propanol).

19. La diméthyl-1,7 isobutyl-3 [(diphénylméthyloxy-4 pipéridino)-3 propyl]-8 xanthine, P.F. du fumarate correspondant: 194°C (éthanol).

20. La méthyl-1 isobutyl-3 (diphénylméthyl-4 pipérazinyl méthyl)-8 xanthine, P.F.: 182°C.

21. La méthyl-1 isobutyl-3 [(diparafluorophénylméthyl-4 pipérazinyl)-2 éthyl]-8 xanthine, P.F.: 200°C.

22. La méthyl-1 isobuyl-3 [(cinnamyl-4 pipérazinyl)-2 éthyl]-8 xanthine, P.F.: 140°C (chlorure de méthylène).

23. L'éthyl-1 isobutyl-3 [(diphénylméthyl-4 pipérazinyl)-3 propyl]-8 xanthine, P.F. (capillaire) du fumarate correspondant: 201-205°C (n. propanol).

24. L'éthyl-1 isobutyl-3 méthyl-7 [(diphénylméthyl-4 pipérazinyl)-3 propyl]-8 xanthine, P.F. du maléate correspondant: 193°C (n. propanol).

25. L'isobutyl-3 [(diphénylméthyl-4 pipérazinyl)-2 éthyl]-8 xanthine, P.F.: 240°C (acétate d'éthyle).

26. La benzyl-3 [(diphénylméthyl-4 pipérazinyl)-2 éthyl]-8 xanthine, P.F. (capillaire): 232-235°C (acétate d'éthyle).

27. La méthyl-1 isobutyl-3 (dihydroxy-2,3 propyl)-7 [(diphénylméthyl-4 pipérazinyl)-2 éthyl]-8 xanthine, P.F. (capillaire) du dichlorhydrate correspondant: 145-155°C (isopropanol/éther).

28. La méthyl-1 isobutyl-3 (hydroxy-2 éthyl)-7 [(diphénylméthyl-4 pipérazine)-2 éthyl]-8 xanthine, P.F. (capillaire) du dichlorhydrate correspondant: 190-200°C (isopropanol/éther).

29. La R,S méthyl-1 isobutyl-3 hydroxy-1 [(diphénylméthyl-4 pipérazinyl)-2 éthyl]-8 xanthine, P.F.: 212°C (éther éthylique).

30. L'isobutyl-3 [hydroxy-1 (diphénylméthyl-4 pipérazinyl)-2 éthyl] xanthine, P.F.: 234°C avec décomposition (acétate d'éthyle).

Les produits objets des exemples 2 à 30 ont été préparés à partir des composés de formule $II_a$ dont les caractéristiques sont regroupées ci-après dans le tableau A. Ces produits de formule $II_a$ ont eux-mêmes été préparés selon le procédé objet dù schéma opératoire donné précédemment, à partir des composés·dont les caractéristiques sont regroupées ci-après dans les tableaux B, C et D.
les caractéristiques sont regroupées ci-après dans le tableau A. Ces produits de formule $II_a$ ont eux-mêmes été préparés selon le procédé objet du schéma opératoire donné précédemment, à partir des composés dont les caractéristiques sont regroupées ci-après dans les tableaux B, C et D.

*Tableau A*

Dérivés de formule

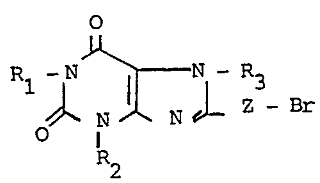

(IIa)

| $R_1$ | $R_2$ | $R_3$ | Z | P.F. (Kofler) (en °C) |
|---|---|---|---|---|
| $CH_3$ | $CH_3$ | H | $(CH_2)_2$ | 227 |

| $R_1$ | $R_2$ | $R_3$ | Z | P.F. (Kofler) (en °C) |
|---|---|---|---|---|
| $CH_3$ | $CH_3$ | H | $(CH_2)_3$ | 220 |
| $CH_3$ | $CH_3$ | $CH_3$ | $(CH_2)_3$ | 130 |
| $CH_3$ | $-CH_2-CH-(CH_3)_2$ | H | $-CH_2$ | 224 |
| $CH_3$ | $-CH_2-CH-(CH_3)_2$ | H | $(CH_2)_2$ | 210 |
| $CH_3$ | $-CH_2-CH-(CH_3)_2$ | H | $(CH_2)_3$ | 182 |
| $CH_3$ | $-CH_2-CH-(CH_3)_2$ | $CH_3$ | $(CH_2)_2$ | 114 |
| $CH_3$ | $-CH_2-CH-(CH_3)_2$ | $CH_3$ | $(CH_2)_3$ | 100 |
| $CH_3$ | $-CH_2-CH_2-CH_3$ | $CH_3$ | $(CH_2)_3$ | 92 |
| $CH_3$ | $-CH_2-CH-(CH_3)_2$ | H | $(CH_2)_3$ | 166 |
| $CH_3$ | 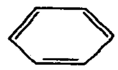 | $CH_3$ | $(CH_2)_3$ | 156 |
| $C_2H_5$ | $-CH_2-CH-(CH_3)_2$ | H | $(CH_2)_3$ | 172 |
| H | $-CH_2$ 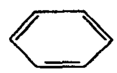 | H | $(CH_2)_2$ | 252 |
| H | $-CH_2-CH-(CH_3)_2$ | H | $(CH_2)_2$ | 292-294 |
| * $CH_3$ | $-CH_2-CH-(CH_3)_2$ | H | $-CH(OH)-CH_2$ | 160 |
| * H | $-CH_2-CH-(CH_3)_2$ | H | $-CH(OH)-CH_2$ | 250 |

N.B.: Les dérivés* ont été préparés par analogie avec G. Ehrhar *et coll.*, Arch. der Pharm. *289*, 453-459 (1956), selon le schéma suivant:

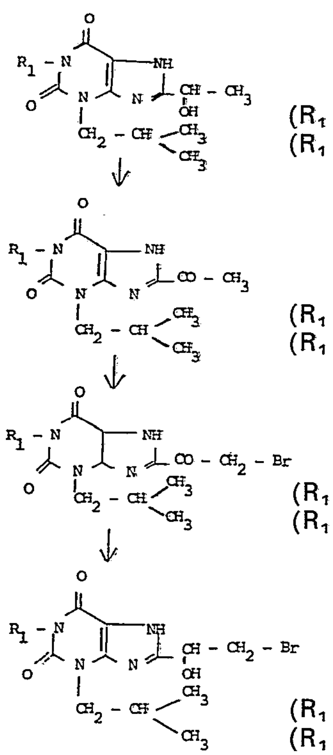

($R_1$ = H, P.F.: 288°C)
($R_1$ = $CH_3$, P.F.: 238°C)

($R_1$ = H, P.F.: 264°C)
($R_1$ = $CH_3$, P.F.: 222°C)

($R_1$ = H, P.F.: 192°C)
($R_1$ = $CH_3$, P.F.: 220°C)

($R_1$ = H, P.F.: 250°C)
($R_1$ = $CH_3$, P.F.: 160°C)

*Tableau B*

Dérivés de formule

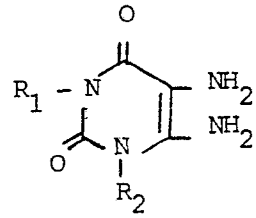

(VI)

| $R_1$ | $R_2$ | P.F. (Kofler) (en °C) |
|---|---|---|
| $CH_3$ | $CH_3$ | 210-214 |
| $CH_3$ | $-CH_2-CH-(CH_3)_2$ | 174 |
| $CH_3$ | $-CH_2-CH=CH_2$ | 116 |
| $CH_3$ | $-CH_2-CH-(CH_3)_2$ | 172 |
| $CH_3$ | 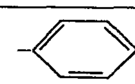 | 250-252 |
| $C_2H_5$ | $-CH_2-CH-(CH_3)_2$ | amorphe |
| H | $-CH_2-$ 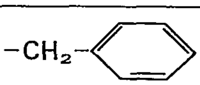 | 243-244 |
| H | $-CH_2-CH-(CH_3)_2$ | non isolé |

*Tableau C*

Dérivés de formule

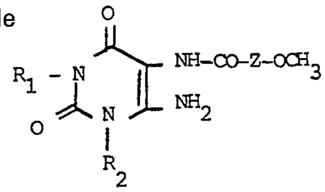

(VII)

| $R_1$ | $R_2$ | Z | P.F. (Kofler) (en ° C) |
|---|---|---|---|
| $CH_3$ | $CH_3$ | $(CH_2)_2$ | 208 |
| $CH_3$ | $CH_3$ | $(CH_2)_3$ | 221 |
| $CH_3$ | $-CH_2-CH-(CH_3)_2$ | $CH_2$ | amorphe |
| $CH_3$ | $-CH_2-CH-(CH_3)_2$ | $(CH_2)_2$ | 200 |
| $CH_3$ | $-CH_2-CH-(CH_3)_2$ | $(CH_2)_3$ | 150 |
| $CH_3$ | $-CH_2-CH=CH_2$ | $(CH_2)_3$ | amorphe |
| $C_2H_5$ | $CH_2-CH-(CH_3)_2$ | $(CH_2)_3$ | amorphe |
| $CH_3$ | 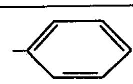 | $(CH_2)_3$ | amorphe |
| H | $-CH_2-$ 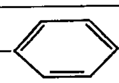 | $(CH_2)_2$ | 190 |
| H | $-CH_2-CH-(CH_3)_2$ | $(CH_2)_2$ | 158 |

*Tableau D*

Dérivés de formule

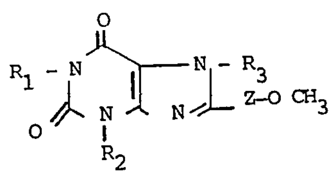

(IX)

| $R_1$ | $R_2$ | $R_3$ | Z | P.F. (Kofler) (en ° C) |
|---|---|---|---|---|
| $CH_3$ | $CH_3$ | H | $(CH_2)_2$ | 198 |
| $CH_3$ | $CH_3$ | H | $(CH_2)_3$ | 192 |
| $CH_3$ | $CH_3$ | $CH_3$ | $(CH_2)_2$ | 156 |
| $CH_3$ | $CH_3$ | $CH_3$ | $(CH_2)_3$ | 96 |
| $CH_3$ | $-CH_2-CH-(CH_3)_2$ | H | $CH_2$ | 196 |
| $CH_3$ | $-CH_2-CH-(CH_3)_2$ | H | $(CH_2)_2$ | 165 |
| $CH_3$ | $-CH_2-CH-(CH_3)_2$ | H | $(CH_2)_3$ | 144 |
| $CH_3$ | $-CH_2-CH-(CH_3)_2$ | $CH_3$ | $(CH_2)_2$ | 82 |
| $CH_3$ | $-CH_2-CH-(CH_3)_2$ | $CH_3$ | $(CH_2)_3$ | 76 |
| $CH_3$ | $-CH_2-CH=CH_2$ | H | $(CH_2)_3$ | 160 |
| $CH_3$ | $-CH_2-CH=CH_2$ | $CH_3$ | $(CH_2)_3$ | 44-45 |
| $CH_3$ | $-CH_2-CH_2-CH_3$* | $CH_3$ | $(CH_2)_3$ | 67 |
| $C_2H_5$ | $-CH_2-CH-(CH_3)_2$ | H | $(CH_2)_3$ | 137-139 |

| $R_1$ | $R_2$ | $R_3$ | Z | P.F. (Kofler) (en °C) |
|---|---|---|---|---|
| $C_2H_5$ | $-CH_2-CH-(CH_3)_2$ | $CH_3$ | $(CH_2)_3$ | 80 |
| $CH_3$ | 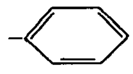 | H | $(CH_2)_3$ | 216 |
| $CH_3$ | 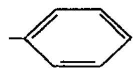 | $CH_3$ | $(CH_2)_3$ | 163 |
| H | $-CH_2-$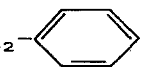 | H | $(CH_2)_2$ | 190 |
| H | $-CH_2-CH-(CH_3)_2$ | H | $(CH_2)_2$ | 250 |

* Ce dérivé propyl-3 a été préparé par réduction du dérivé allyl-3 correspondant, sous une pression d'hydrogène de 6 atmosphères en présence de nickel comme catalyseur.

N.B.: Les dérivés de formule VI sont préparés selon le schéma opératoire ci-après:

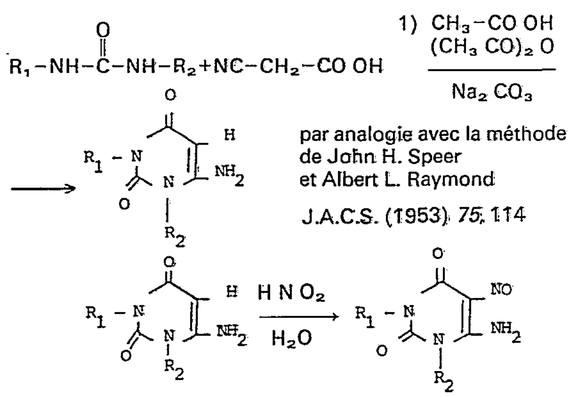


par analogie avec la méthode de G.N. Krutovskikh et coll., Pharmaceutical Chemistry Journal (1977) *11*(2), 224.

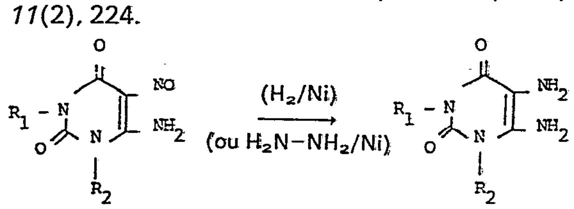


*Exemple 31:*

Méthyl-1 isobutyl-3 (hydroxy-2 éthyl)-7 [(diphénylméthyl-4 pipérazinyl)-2 éthyl]-8 xanthine:

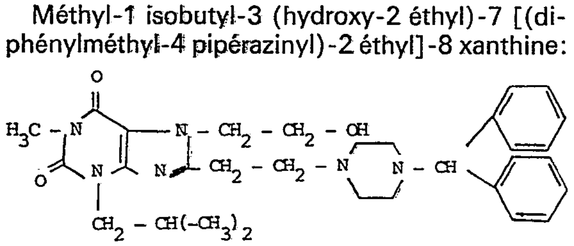

10 g de méthyl-1 isobutyl-3 [(diphénylméthyl-4 pipérazinyl)-2 éthyl]-8 xanthine et 37,6 g de carbonate de potassium sont agités dans 200 ml de diméthylformamide et portés à 100°C. On ajoute rapidement 10 ml de chlorhydrine du glycol et agite 1 h à 110°C. On ajoute ensuite rapidement 20 ml de chlorhydrique du glycol et maintient 1 h à 110°C sous agitation, on ajoute ensuite 40 g de carbonate de potassium, puis 20 ml de chlorhydrine du glycol. L'ensemble est maintenu à 110°C pendant 3½ h, puis à température ambiante pendant 72 h. Le mélange est alors concentré à sec, le résidu est repris dans un mélange de chlorure de méthylène et d'eau. La phase organique est séchée sur sulfate de sodium puis concentrée à sec. On effectue une chromatographie sur 750 g de silice (0,04-0,063 mm). On élue d'abord, au chlorure de méthylène, puis à l'acétate d'éthyle pur, ensuite avec un mélange acétate d'éthyle-méthanol (95-5) et enfin avec un mélange acétate d'éthyle-méthanol (90-10).

On obtient 8,6 g de base que l'on dissout dans 50 ml d'isopropanol. On ajoute suffisamment d'éther chlorhydrique pour avoir un milieu légèrement acide. On précipite le chlorhydrate du produit cherché avec un gros excès d'éther anhydre. On essore et lave à l'éther. Après séchage, à 115°C sous 0,6 mmHg, on obtient 8,7 g de dichlorhydrate de méthyl-1 isobutyl-3 (hydroxy-2 éthyl)-7 [(diphénylméthyl-4 pipérazinyl)-2 éthyl]-8 xanthine, P.F. (capillaire): 190-200°C (isopropanol/éther). Rendement: 71%.

*Exemple 32:*

De la même façon a été préparée la méthyl-1 isobutyl-3 (dihydroxy-2,3 propyl)-7 [(diphénylméthyl-4 pipérazinyl)-2 éthyl]-8 xanthine, dont le dichlorhydrate fond (capillaire) à 145-155°C (isopropanol/éther).

**Revendications** pour les Etats contractants BE, CH, DE, FR, GB, IT, LI, LU, NL, SE

1. Les dérivés de la xanthine de formule générale:

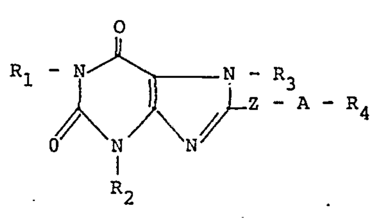

(I)

dans laquelle:

$R_1$ représente un atome d'hydrogène ou un radical alkyle, en chaîne droite ou ramifiée, renfermant de 1 à 5 atomes de carbone;

$R_2$ représente un radical hydrocarboné renfermant jusqu'à 6 atomes de carbone, en chaîne droite ou ramifiée et comportant éventuellement une double liaison, un radical phényle, ou un radical benzyle;

$R_3$ représente un atome d'hydrogène ou un radical alkyle, hydroxyalkyle ou dihydroxyalkyle renfermant chacun de 1 à 5 atomes de carbone;

$R_4$ représente un groupe de formule:

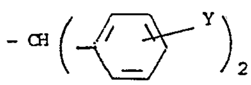 ou 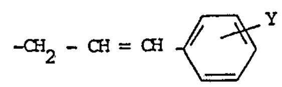

dans lesquelles:

Y représente un atome d'hydrogène ou d'halogène ou un radical alkyle ou alcoxy renfermant chacun de 1 à 5 atomes de carbone, ou un radical hydroxyle;

Z représente un radical méthylène ou un radical hydrocarboné en chaîne droite ou ramifiée, ayant de 2 à 5 atomes de carbone, éventuellement substitué par un radical hydroxyle, et

A représente un reste aminé de type:

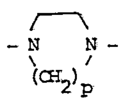

dans lequel p prend les valeurs 2 ou 3, ou

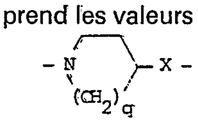

dans lequel:

q prend les valeurs 1, ou 2, et

X représente une liaison simple, un atome d'oxygène ou un groupe:

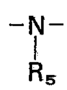

dans lequel:

$R_5$ représente un atome d'hydrogène ou un radical alkyle ou alkylène ayant chacun de 1 à 5 atomes de carbone, ou

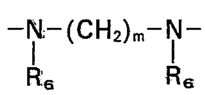

dans lequel:

m représente un nombre entier de 2 à 6, et

$R_6$ représente un radical alkyle renfermant de 1 à 5 atomes de carbone.

2. Les sels des composés de la revendication 1 avec des acides appropriés.

3. Les sels selon la revendication 2 qui sont physiologiquement tolérables.

4. La méthyl-1 isobutyl-3 [(diphénylméthyl-4 pipérazinyl-2 éthyl]-8 xanthine.

5. La méthyl-1 isobutyl-3 [(diphénylméthyl-4 pipérazinyl)-3 propyl]-8 xanthine.

6. La diméthyl-1,7 isobutyl-3 [(diphénylméthyl-4 pipérazinyl)-3 propyl]-8 xanthine.

7. La méthyl-1 isobutyl-3 [(diparafluorophénylméthyl-4 pipérazinyl)-2 éthyl]-8 xanthine.

8. La diméthyl-1,7 isobutyl-3 [(diparafluorophényl-méthyl-4 pipérazinyl)-3 propyl]-8 xanthine.

9. La méthyl-1 isobutyl-3 [(diphénylméthyloxy-4 pipéridino)-2 éthyl]-8 xanthine.

10. La diméthyl-1,7 isobutyl-3 [(diphénylméthyloxy-4 pipéridino)-3 propyl]-8 xanthine.

11. La méthyl-1 isobutyl-3 [(diparafluorophénylméthyl-4 pipérazinyl)-2 éthyl]-8 xanthine.

12. L'isobutyl-3 [(diphénylméthyl-4 pipérazinyl)-2 éthyl]-8 xanthine.

13. La méthyl-1 isobutyl-3 (dihydroxy-2,3 propyl)-7 [(diphénylméthyl-4 pipérazinyl)-2 éthyl]-8 xanthine.

14. Le procédé de préparation des composés de la revendication 1, caractérisé en ce que l'on condense un dérivé halogéné de formule générale II:

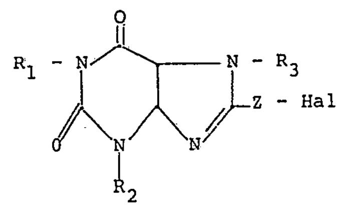

(II)

dans laquelle:

$R_1$, $R_2$, $R_3$ et Z ont les significations définies dans la revendication 1, et

Hal représente un atome de chlore ou de brome, avec un dérivé aminé de formule générale III:

$$H-A-R_4 \quad (III)$$

dans laquelle:

A et $R_4$ ont les significations énoncées dans la revendication 1.

15. Le procédé de préparation de composés de la revendication 1, répondant à la formule générale I':

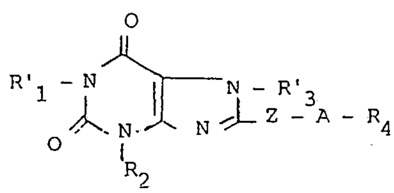

(I')

dans laquelle:

$R_2$, $R_4$, Z et A ont les significations définies dans la revendication 1;

$R'_1$ représente un radical alkyle en chaîne droite ou ramifiée renfermant de 1 à 5 atomes de carbone, et

$R'_3$ représente un radical alkyle, hydroxyalkyle ou dihydroxyalkyle contenant chacun de 1 à 5 atomes de carbone,

caractérisé en ce que l'on condense un dérivé de formule générale II':

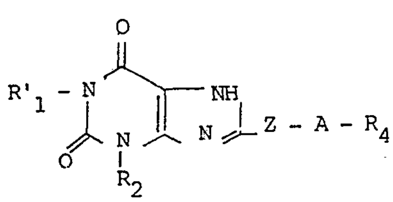

(II')

dans laquelle:

R'$_1$, $R_2$, $R_4$, Z et A sont tels que précédemment définis, avec un dérivé halogéné de formule générale III':

$$R'_3-X \qquad (III')$$

dans laquelle:

R'$_3$ a la signification énoncée précédemment, et X représente un atome de chlore ou de brome.

16. Les compositions pharmaceutiques contenant comme principe actif un composé selon les revendications 1 ou 3 à 13, avec les excipients pharmaceutiques appropriés.

**Revendications** pour l'Etat contractant: AT

1. Procédé de préparation des dérivés de la xanthine de formule générale:

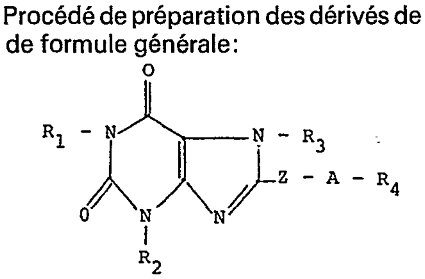

(I)

dans laquelle:

$R_1$ représente un atome d'hydrogène ou un radical alkyle, en chaîne droite ou ramifiée, renfermant de 1 à 5 atomes de carbone;

$R_2$ représente un radical hydrocarboné renfermant jusqu'à 6 atomes de carbone, en chaîne droite ou ramifiée et comportant éventuellement une double liaison, un radical phényle, ou un radical benzyle;

$R_3$ représente un atome d'hydrogène ou un radical alkyle, hydroxyalkyle ou dihydroxyalkyle renfermant chacun de 1 à 5 atomes de carbone;

$R_4$ représente un groupe de formule:

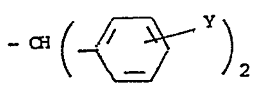 ou 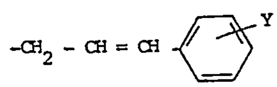

dans lesquelles:

Y représente un atome d'hydrogène ou d'halogène, un radical alkyle ou alkoxy renfermant chacun de 1 à 5 atomes de carbone, ou un radical hydroxyle;

Z représente un radical méthylène ou un radical hydrocarboné en chaîne droite ou ramifiée, ayant de 2 à 5 atomes de carbone, éventuellement substitué par un radical hydroxyle, et

A représente un reste aminé de type:

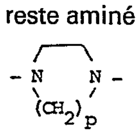

dans lequel p prend les valeurs 2 ou 3, ou

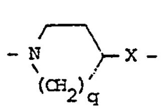

dans lequel:

q prend les valeurs 1 ou 2, et

X représente une liaison simple, un atome d'oxygène ou un groupe:

$$-\underset{\substack{|\\ R_5}}{N}-$$

dans lequel:

$R_5$ représente un atome d'hydrogène ou un radical alkyle ou alkylène ayant chacun de 1 à 5 atomes de carbone, ou

$$-\underset{\substack{|\\ R_6}}{N}-(CH_2)_m-\underset{\substack{|\\ R_6}}{N}-$$

dans lequel:

m représente un nombre entier de 2 à 6, et

$R_6$ représente un radical alkyle renfermant de 1 à 5 atomes de carbone,

et de leurs sels d'addition avec des acides appropriés, caractérisé en ce que l'on condense un dérivé halogéné de formule générale II:

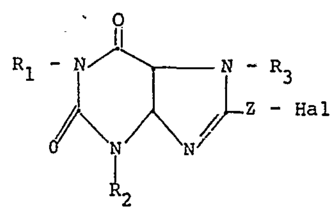

(II)

dans laquelle:

$R_1$, $R_2$, $R_3$ et Z ont les significations ci-dessus définies, et

Hal représente un atome de chlore ou de brome, avec un dérivé aminé de formule générale III:

$$H-A-R_4 \qquad (III)$$

dans laquelle:

A et $R_4$ ont les significations énoncées précédemment, et si on le désire on traite les dérivés I ainsi obtenus avec des acides appropriés pour donner les sels d'addition correspondants.

2. Le procédé de préparation des dérivés de la xanthine répondant à la formule générale I':

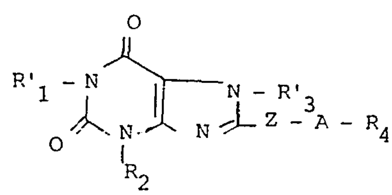

(I')

dans laquelle:

$R_2$, $R_4$, Z et A ont les significations définies dans la revendication 1;

R'$_1$ représente un radical alkyle en chaîne droite ou ramifiée renfermant de 1 à 5 atomes de carbone, et

R'$_3$ représente un radical alkyle, hydroxyalkyle ou dihydroxyalkyle contenant chacun de 1 à 5 atomes de carbone,

et de leur sels d'addition avec des acides appropriés, caractérisé en ce que l'on condense un dérivé de formule générale II':

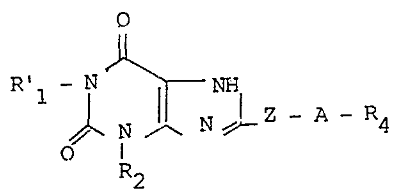 (II')

dans laquelle:

$R'_1$, $R_2$, $R_4$, Z et A sont tels que précédemment définis,

avec un dérivé halogéné de formule générale III':

$$R'_3-X \qquad (III')$$

dans laquelle:

$R'_3$ a la signification énoncée précédemment, et

X représente un atome de chlore ou de brome;

et si on le désire on traite les dérivés I' ainsi obtenus avec des acides appropriés pour donner les sels d'addition correspondants.

**Patentansprüche** für die Vertragsstaaten BE, CH, DE, FR, GB, IT, LI, LU, NL, SE

1. Xanthinderivate der allgemeinen Formel:

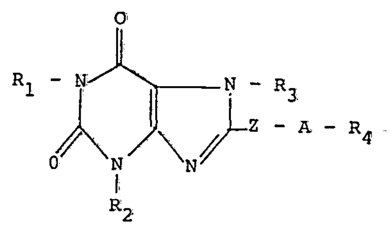 (I)

in der:

$R_1$ ein Wasserstoffatom oder eine geradkettige oder verzweigte Alkylgruppe mit 1 bis 5 Kohlenstoffatomen;

$R_2$ eine geradkettige oder verzweigte und gegebenenfalls eine Doppelbindung aufweisende Kohlenwasserstoffgruppe mit bis zu 6 Kohlenstoffatomen, eine Phenylgruppe oder eine Benzylgruppe;

$R_3$ ein Wasserstoffatom oder eine Alkylgruppe, Hydroxylalkylgruppe oder Dihydroxyalkylgruppe mit jeweils 1 bis 5 Kohlenstoffatomen;

$R_4$ eine Gruppe der Formeln:

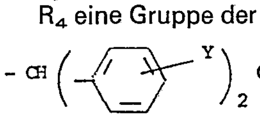 oder 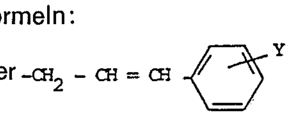

worin:

Y für ein Wasserstoffatom, ein Halogenatom, eine Alkyl- oder Alkoxygruppe mit jeweils 1 bis 5 Kohlenstoffatomen oder eine Hydroxylgruppe steht;

Z eine Methylengruppe oder eine geradkettige oder verzweigte, gegebenenfalls mit einer Hydroxylgruppe substituierte Kohlenwasserstoffgruppe mit 2 bis 5 Kohlenstoffatomen, und

A einen stickstoffhaltigen Rest der Formel:

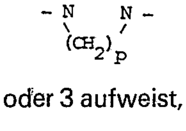

in der:

p Werte von 2 oder 3 aufweist, oder der Formel:

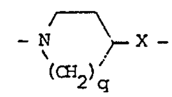

in der:

q Werte von 1 oder 2 aufweist, und

X für eine Einfachbindung, ein Sauerstoffatom oder eine Gruppe der Formel

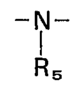

steht, worin:

$R_5$ ein Wasserstoffatom oder eine Alkyl- oder Alkylengruppe mit jeweils 1 bis 5 Kohlenstoffatomen darstellt, oder der Formel:

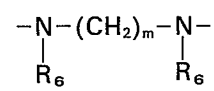

in der:

m für eine ganze Zahl mit einem Wert von 2 bis 6, und

$R_6$ für eine Alkylgruppe mit 1 bis 5 Kohlenstoffatomen stehen, bedeuten.

2. Die Salze der Verbindungen nach Anspruch 1 mit geeigneten Säuren.

3. Die Salze nach Anspruch 2, die physiologisch verträglich sind.

4. 1-Methyl-3-isobutyl-8 [2-(4-diphenylmethyl-piperazinyl)-ethyl] xanthin.

5. 1-Methyl-3-isobutyl-8 [3-(4-diphenylmethyl-piperazinyl)-propyl] xanthin.

6. 1,7-Dimethyl-3-isobutyl-8 [3-(4-diphenylmethyl-piperazinyl)-propyl] xanthin.

7. 1-Methyl-3-isobutyl-8 [2-(4-di-p-fluorphenylmethyl-piperazinyl)-ethyl] xanthin.

8. 1,7-Dimethyl-3-isobutyl-8 [3-(4-di-p-fluorphenylmethyl-piperazinyl)-propyl] xanthin.

9. 1-Methyl-3-isobutyl-8 [2-(4-diphenylmethyloxy-piperidino)-ethyl] xanthin.

10. 1,7-Dimethyl-3-isobutyl-8 [3-(4-diphenylmethyloxy-piperidino)-propyl] xanthin.

11. 1-Methyl-3-isobutyl-8 [2-(4-di-p-fluorphenylmethyl-piperazinyl)-ethyl] xanthin.

12. 3-Isobutyl-8 [2-(4-diphenylmethyl-piperazinyl)-ethyl] xanthin.

13. 1-Methyl-3-isobutyl-7-(2,3-dihydroxypropyl)-8 [2-(4-diphenylmethyl-piperazinyl)-ethyl] xanthin.

14. Verfahren zur Herstellung der Verbindungen nach Anspruch 1, dadurch gekennzeichnet, dass man ein Halogenderivat der allgemeinen Formel II:

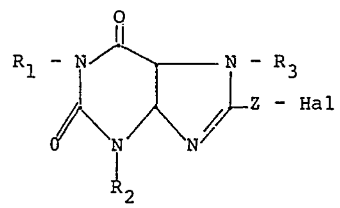 (II)

in der:

$R_1$, $R_2$, $R_3$ und Z die in Anspruch 1 angegebenen Bedeutungen besitzen, und

Hal ein Chloratom oder Bromatom bedeutet,

mit einem Aminderivat der allgemeinen Formel III:

$$H-A-R_4 \quad (III)$$

in der:

A und $R_4$ die in Anspruch 1 angegebenen Bedeutungen besitzen, kondensiert.

15. Verfahren zur Herstellung von Verbindungen nach Anspruch 1 der allgemeinen Formel I':

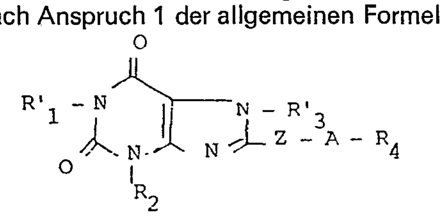

(I')

in der:

$R_2$, $R_4$, Z und A die in Anspruch 1 angegebenen Bedeutungen besitzen, und

$R'_1$ für eine geradkettige oder verzweigte Alkylgruppe mit 1 bis 5 Kohlenstoffatomen, und

$R'_3$ für eine Alkylgruppe, Hydroxyalkylgruppe oder Dihydroxylalkylgruppe mit jeweils 1 bis 5 Kohlenstoffatomen stehen,

dadurch gekennzeichnet, dass man ein Derivat der allgemeinen Formel II':

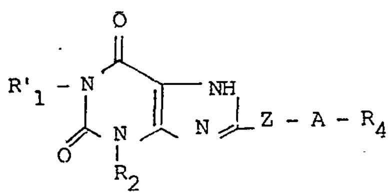

(II')

in der:

$R'_1$, $R_2$, $R_4$, Z und A die oben angegebenen Bedeutungen besitzen, mit einem Halogenderivat der allgemeinen Formel III':

$$R'_3-X \quad (III')$$

in der:

$R'_3$ die oben angegebenen Bedeutungen besitzt, und

X ein Chloratom oder ein Bromaton darstellt, kondensiert.

16. Pharmazeutische Zubereitungen enthaltend als Wirkstoff eine Verbindung nach einem der Ansprüche 1 oder 3 bis 13 mit geeigneten pharmazeutischen Trägermaterialien.

**Patentansprüche** für den Vertragsstaat: AT

1. Verfahren zur Herstellung von Xanthinverbindungen der allgemeinen Formel I:

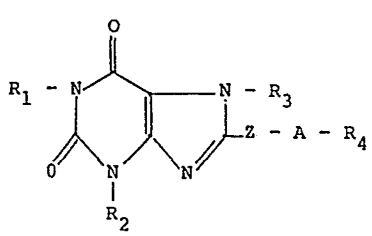

(I)

in der:

$R_1$ ein Wasserstoffatom oder eine geradkettige oder verzweigte Alkylgruppe mit 1 bis 5 Kohlenstoffatomen;

$R_2$ eine geradkettige oder verzweigte und gegebenenfalls eine Doppelbindung aufweisende Kohlenwasserstoffgruppe mit bis zu 6 Kohlenstoffatomen, eine Phenylgruppe oder eine Benzylgruppe;

$R_3$ ein Wasserstoffatom oder eine Alkylgruppe, Hydroxyalkylgruppe oder Dihydroxyalkylgruppe mit jeweils 1 bis 5 Kohlenstoffatomen;

$R_4$ eine Gruppe der Formeln:

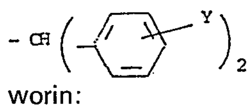 oder 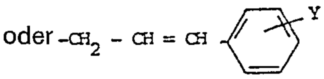

worin:

Y für ein Wasserstoffatom, ein Halogenatom, eine Alkyl- oder Alkoxygruppe mit jeweils 1 bis 5 Kohlenstoffatomen oder eine Hydroxylgruppe steht;

Z eine Methylengruppe oder eine geradkettige oder verzweigte, gegebenenfalls mit einer Hydroxylgruppe substituierte Kohlenwasserstoffgruppe mit 2 bis 5 Kohlenstoffatomen, und

A einen stickstoffhaltigen Rest der Formel:

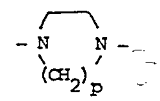

in der:

p Werte von 2 oder 3 aufweist, oder der Formel:

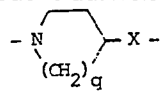

in der:

q Werte von 1 oder 2 aufweist, und

X für eine Einfachbindung, ein Sauerstoffatom oder eine Gruppe der Formel:

$$-\underset{\displaystyle R_5}{\underset{|}{N}}-$$

steht, worin $R_5$ ein Wasserstoffatom oder eine Alkyl- oder Alkylengruppe mit jeweils 1 bis 5 Kohlenstoffatomen darstellt, oder der Formel:

$$-\underset{\displaystyle R_6}{\underset{|}{N}}-(CH_2)_m-\underset{\displaystyle R_6}{\underset{|}{N}}-$$

in der:

m für eine ganze Zahl mit einem Wert von 2 bis 6, und

$R_6$ für eine Alkylgruppe mit 1 bis 5 Kohlenstoffatomen stehen, bedeuten, sowie deren Additionssalze mit geeigneten Säuren, dadurch gekennzeichnet, dass man ein Halogenderivat der allgemeinen Formel II:

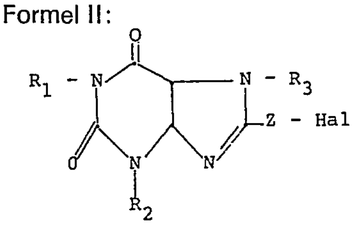

(II)

in der:

$R_1$, $R_2$, $R_3$ und Z die oben angegebenen Bedeutungen besitzen, und

Hal für ein Chloratom oder ein Bromatom steht, mit einem Aminderivat der allgemeinen Formel III:

$$H-A-R_4 \quad (III)$$

in der:

A und $R_4$ die oben angegebenen Bedeutungen besitzen, kondensiert; und gewünschtenfalls die in dieser Weise erhaltenen Derivate I mit geeigneten Säuren zur Bildung der entsprechenden Additionssalze behandelt.

2. Verfahren zur Herstellung von Xanthinderivaten der allgemeinen Formel I':

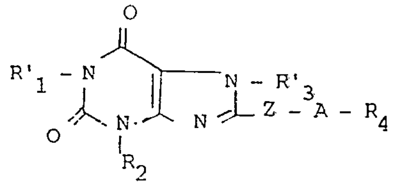

(I')

in der:

$R_2$, $R_4$, Z und A die in Anspruch 1 angegebenen Bedeutungen besitzen, und

$R'_1$ für eine geradkettige oder verzweigte Alkylgruppe mit 1 bis 5 Kohlenstoffatomen, und

$R'_3$ für eine Alkylgruppe, Hydroxylalkylgruppe oder Dihydroxylalkylgruppe, die jeweils 1 bis 5 Kohlenstoffatome aufweisen, stehen,

und deren Additionssalze mit geeigneten Säuren, dadurch gekennzeichnet, dass man ein Derivat der allgemeinen Formel II':

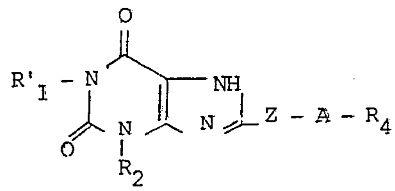

(II')

in der:

$R'_1$, $R_2$, $R_4$, Z und A die oben angegebenen Bedeutungen besitzen,

mit einem Halogenderivat der allgemeinen Formel III':

$$R'_3{-}X \qquad (III')$$

in der:

$R'_3$ die oben angegebenen Bedeutungen besitzt, und

X für ein Chloratom oder ein Bromatom steht, kondensiert; und gewünschtenfalls die in dieser Weise erhaltenen Derivate I' mit geeigneten Säuren zur Bildung der entsprechenden Additionssalze behandelt.

**Claims** for the contracting states BE, CH, DE, FR, GB, IT, LI, LU, NL, SE

1. Xanthine derivatives of the general formula I:

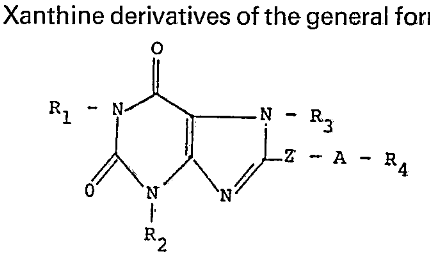

(I)

in which:

$R_1$ represents a hydrogen atom or a straight-chain or branched alkyl radical containing from 1 to 5 carbon atoms;

$R_2$ represents a straight-chain or branched hydrocarbon radical containing up to 6 carbon atoms and optionally having a double bond, or represents a phenyl radical or a benzyl radical;

$R_3$ represents a hydrogen atom or an alkyl, hydroxyalkyl or dihydroxylalkyl radical each containing from 1 to 5 carbon atoms;

$R_4$ represents a group of the formula:

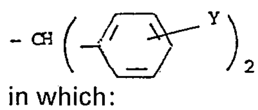 or 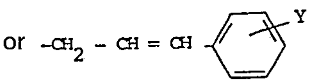

in which:

Y represents a hydrogen or halogen atom or an alkyl or alkoxy radical each containing from 1 to 5 carbon atoms, or a hydroxy radical;

Z represents a methylene radical or a straight-chain or branched hydrocarbon radical having from 2 to 5 carbon atoms which is optionally substituted by a hydroxy radical, and

A represents an amine radical of the type:

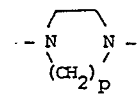

in which:

p has the value 2 or 3, or

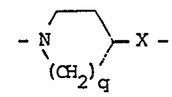

in which:

q has the value 1 or 2, and

X represents a single bond, an oxygen atom or a group:

$$-\underset{\displaystyle R_5}{\underset{|}{N}}-$$

in which:

$R_5$ represents a hydrogen atom or an alkyl or alkenyl radical each having from 1 to 5 carbon atoms, or

$$-\underset{\displaystyle R_6}{\underset{|}{N}}-(CH_2)_m-\underset{\displaystyle R_6}{\underset{|}{N}}-$$

in which:

m represents an integer from 2 to 6, and

$R_6$ represents an alkyl radical containing from 1 to 5 carbon atom.

2. The salts of the compounds of Claim 1 with suitable acids.

3. The salts according to Claim 2 that are physiologically tolerable.

4. 1-Methyl-3-isobutyl-8 [2-(4-diphenylmethyl-piperazinyl)-ethyl] xanthine.

5. 1-Methyl-3-isobutyl-8 [3(4-diphenylmethyl-piperazinyl)-propyl] xanthine.

6. 1,7-Dimethyl-3-isobutyl-8 [3-(4-diphenylmethyl-piperazinyl)-propyl] xanthine.

7. 1-Methyl-3-isobutyl-8 [2-(4-di-para-fluorophenylmethyl-piperazinyl)-ethyl] xanthine.

8. 1,7-Dimethyl-3-isobutyl-8 [3-(4-di-para-fluorophenylmethyl-piperazinyl)-propyl] xanthine.

9. 1-Methyl-3-isobutyl-8 [2-(4-diphenylmethyloxy-piperidino)-ethyl] xanthine.

10. 1,7-Dimethyl-3-isobutyl-8 [3-(4-diphenylmethyloxy-piperidino)-propyl] xanthine.

11. 1-Methyl-3-isobutyl-8 [2-(4-di-para-fluorophenylmethyl-piperazinyl)-ethyl] xanthine.

12. 3-Isobutyl-8 [2-(4-diphenylmethyl-piperazinyl)-ethyl] xanthine.

13. 1-Methyl-3-isobutyl-7-(2,3-dihydroxypropyl)-8 [2-(4-diphenylmethyl-piperazinyl)-ethyl] xanthine.

14. Process for the preparation of the compounds of Claim 1, characterised in that a halogen derivative of the general formula II:

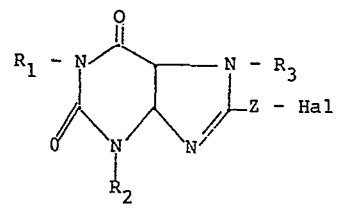

(II)

in which:

$R_1$, $R_2$, $R_3$ and Z have the meanings defined in Claim 1, and

Hal represents a chlorine or bromine atom is condensed with an amine derivative of the general formula III:

$$H-A-R_4 \qquad (III)$$

in which:

A and $R_4$ have the meanings given in Claim 1.

15. Process for the preparation of the compounds of Claim 1 corresponding to the general formula I':

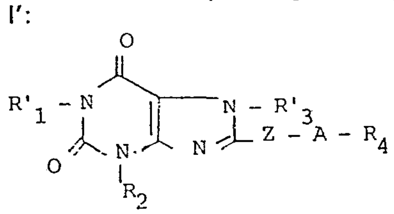

(I')

in which:

$R_2$, $R_4$, Z and A have the meanings defined in Claim 1;

$R'_1$ represents a straight-chain or branched alkyl radical containing from 1 to 5 carbon atoms, and

$R'_3$ represents an alkyl, hydroxyalkyl or dihydroxyalkyl radical each containing from 1 to 5 carbon atoms,

characterised in that a derivative of the general formula II':

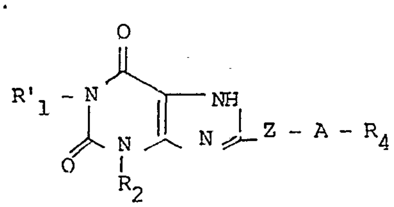

(II')

in which:

$R'_1$, $R_2$, $R_4$, Z and A have the meanings defined above,

is condensed with a halogen derivative of the general formula III':

$$R'_3-X \qquad (III')$$

in which:

$R'_3$ has the meaning given above and X represents a chlorine or bromine atom.

16. Pharmaceutical composition containing as active ingredient a compound according to Claims 1 or 3 to 13 with suitable pharmaceutical excipients.

**Claims** for the contracting state: AT

1. Process for the preparation of xanthine derivatives of the general formula I:

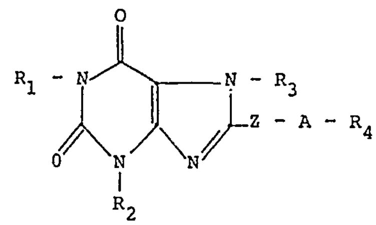

(I)

in which:

$R_1$ represents a hydrogen atom or a straight-chain or branched alkyl radical containing from 1 to 5 carbon atoms;

$R_2$ represents a straight-chain or branched hydrocarbon radical containing up to 6 carbon atoms and optionally having a double bond, or represents a phenyl radical or a benzyl radical;

$R_3$ represents a hydrogen atom or an alkyl, hydroxyalkyl or dihydroxyalkyl radical each containing from 1 to 5 carbon atoms;

$R_4$ represents a group of the formula:

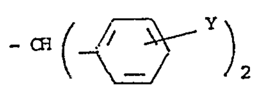 or 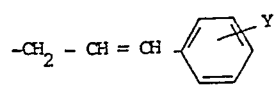

in which:

Y represents a hydrogen or halogen atom or an alkyl or alkoxy radical each containing from 1 to 5 carbon atoms, or a hydroxy radical;

Z represents a methylene radical or a straight-chain or branched hydrocarbon radical having from 2 to 5 carbon atoms which is optionally substituted by a hydroxy radical, and

A represents an amine radical of the type:

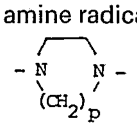

in which:

p has the value 2 or 3; or

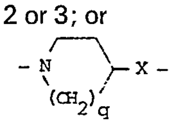

in which:

q has the value 1 or 2, and

X represents a single bond, an oxygen atom or a group:

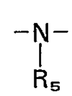

in which:

$R_5$ represents a hydrogen atom or an alkyl or alkenyl radical each having from 1 to 5 carbon atoms; or

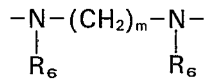

in which:

m represents an integer from 2 to 6, and

$R_6$ represents an alkyl radical containing from 1 to 5 carbon atoms,

and their addition salts with suitable acids, characterised in that a halogen derivative of the general formula II:

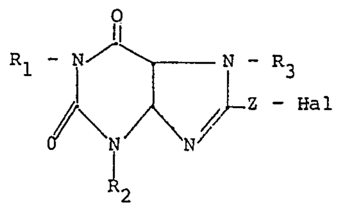

(II)

in which:

$R_1$, $R_2$, $R_3$ and Z have the meanings defined above, and

Hal represents a chlorine or bromine atom

is condensed with an amine derivative of the general formula III:

$$H-A-R_4 \qquad (III)$$

in which:

A and $R_4$ have the meanings given above, and, if desired, the derivatives I so obtained are treated with suitable acids to give the corresponding addition salts.

2. Process for the preparation of the xanthine derivatives corresponding to the general formula I':

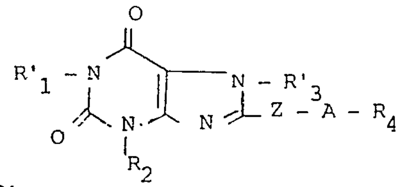

(I')

in which:

$R_2$, $R_4$, Z and A have the meanings defined in Claim 1;

$R'_1$ represents a straight-chain or branched alkyl radical containing from 1 to 5 carbon atoms, and

$R'_3$ represents an alkyl, hydroxylakly or dihydroxyalkyl radical each containing from 1 to 5 carbon atoms,

and their addition salts with suitable acids, characterised in that a derivative of the general formula II':

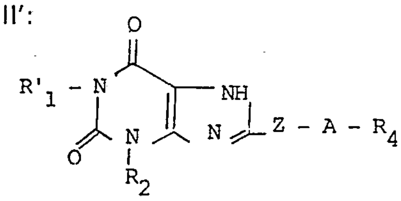

(II')

in which:

$R'_1$, $R_2$, $R_4$, Z and A have the meanings defined above,

is condensed with a halogen derivative of the general formula III':

$$R'_3-X \qquad (III')$$

in which:

$R'_3$ has the meaning given above and X represents a chlorine or bromine atom; and, if desired, the derivatives I' so obtained are treated with suitable acids to give the corresponding addition salts.